# EUROPEAN PATENT APPLICATION

(11) **EP 4 397 334 A1**
(43) Date of publication of application: **10.07.2024**
(21) Application number: 23219152.8
(22) Date of filing: 21.12.2023
(51) Int. Cl.: A61M 1/02, A61M 1/36

(54) **SYSTEM AND METHOD FOR REMOVAL OF FLUID FROM A BIOLOGICAL FLUID FILTER**

(30) Priority: 04.01.2023 US 202363478392 P
(71) Applicant: Fenwal, Inc., Lake Zurich, IL 60047 (US)
(72) Inventor: KUSTERS, Benjamin E., Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

Systems and methods are provided to recover residual biological fluid from a soft sided fluid filter having an inlet and an outlet and which is used by a biological fluid processing system, including a reusable hardware unit comprising a fluid filter compression assembly. The fluid filter compression assembly further includes a housing having an outer wall that defines a housing space that receives the fluid filter, at least one bladder positioned within the housing space, at least one conduit, at least one pump, wherein the at least one conduit is in fluid communication with the at least one bladder and the at least one pump, and at least one clamp configured to selectively stop flow through the fluid filter inlet.

## Description

### Field of the Disclosure

The present subject matter relates to systems and methods for processing a biological fluid, such as blood, blood constituents, or other suspensions of cellular material. More particularly, the present subject matter relates to a system and method to recover residual fluid from a fluid filter within a biological fluid processing system.

### Description of Related Art

Various biological fluid processing systems permit collection of constituents of the fluid being processed, whether fully automated or involving some manual operator intervention. For example, many blood processing systems make it possible to collect blood constituents from a blood source, such as a human donor or patient, or previously collected blood. Such systems may utilize separation of blood constituents and filtration. Typically, in such systems, whole blood (WB) is drawn from a blood source, the particular blood component or constituent is separated, removed and collected, and the remaining blood constituents are returned to the blood source or separately collected. Removing only particular constituents is advantageous when the blood source is a donor, because potentially less time is needed for the donor's body to return to normal or pre-donation levels. Also, donations of particular blood components or constituents may be made at more frequent intervals than when WB is collected in full. This increases the overall supply of blood constituents, such as plasma and platelets, made available for transfer and/or therapeutic treatment or health care.

In practice, it has been common that upon completion of a blood filtration process, some fluid would be left behind and discarded, such as WB or Red Blood Cells (RBCs). The residual fluid loss in a filter, for example, decreased the RBC recovery. For WB filtration, this caused a loss of valuable plasma that could have been collected during the subsequent separation process of the WB unit. This is disadvantageous as plasma is valuable, given that it may be sold to fractionation per ml.

Fluid loss during WB filtration, in particular of plasma, caused the blood industry to move away from filtration of WB units over the past decade in an effort to maximize plasma collection volumes. The industry moved to RBC product leukofiltration, after WB separation. This has proven to be more effective for the current WB manufacturing methods.

### Summary

It is respectfully believed that some systems, such as for example the Configurable System To Automate Blood Component Manufacturing Process, described in International Pub. No. WO 2021/194824, which is incorporated herein by reference in its entirety, may benefit from WB leukofiltration. This is because the platelets are sequestered in the leukofilter in addition to the WBCs. This would benefit the WB automation system because the centrifuge would no longer be required to ensure platelets do not exit with the plasma, since the platelets will have been removed in the filter prior to the WB entering the centrifuge. In turn, this would enable more aggressive plasma separation and larger product volumes without the risk of contaminating the plasma with platelets. However, the residual fluid loss in a filter still results in disadvantageous loss of valuable constituents of the biological fluid being processed.

In order to overcome this disadvantage, the present disclosure provides a system and method to recover residual fluid from a fluid filter within a biological fluid processing system, such as the aforementioned system described in International Pub. No. WO 2021/194824. Thus, the residual fluid that currently would remain in a filter is recovered and sent to the centrifuge to prevent the system from losing plasma to the filter. The present disclosure provides systems and methods that aim to recover the residual fluid that is currently left behind in biological fluid filters, such as leukoreduction filters, by compressing or squeezing the fluid filter upon completion of the subject filtration, such as WB filtration. The invention is not limited to leukofilters or WB filtration and may be applied to evacuate fluid from any type of filtration system utilizing a soft sided or soft shell compressible fluid filter. Various embodiments may be employed, depending on the filter construction, size, and inlet/out port locations or the type of fluid present.

Whole blood typically may be separated into its constituents through various forms of separation, such as by use of centrifugation, a spinning membrane separator or suitable alternatives. This requires that the whole blood be passed through the separator after it is withdrawn from, and before it is returned to, the blood source. To reduce contamination and possible infection, if the blood source is a donor or patient, the blood preferably is contained and processed within a disposable, sealed, sterile fluid flow circuit or disposable kit during the entirety of the processing. Thus, disposable flow circuits include a separation chamber portion, which an operator installs in a durable, reusable hardware, which may include, for example, a separator, drive system, pumps, valve actuators, programmable controller, and the like. The biological fluid, such as WB, however, makes actual contact only with the disposable fluid flow circuit, which assembly is used only once and then discarded.

There are several aspects of the present subject matter which may be embodied separately or together in the devices and systems described and claimed herein. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In a first aspect, the present disclosure provides a system to recover residual biological fluid from a soft sided fluid filter having an inlet and an outlet and which is used by a biological fluid processing system, comprising a reusable hardware unit comprising a fluid filter compression assembly. The fluid filter compression assembly further comprising a housing comprising an outer wall that defines a housing space that receives the fluid filter, at least one bladder positioned within the housing space, at least one conduit, at least one pump, wherein the at least one conduit is in fluid communication with the at least one bladder and the at least one pump, and a clamp configured to selectively stop flow through the fluid filter inlet.

In a second aspect, the present disclosure provides a method of recovering residual biological fluid from a soft sided fluid filter having an inlet and an outlet and which is used by a biological fluid processing system, the method comprising providing a system as set forth in the aforementioned first aspect, passing fluid through the fluid filter inlet, the fluid filter and the fluid filter outlet, engaging the clamp to selectively stop flow through the fluid filter inlet, and engaging the at least one pump to pump fluid media into the at least one bladder, causing the fluid filter to be compressed and to expel residual biological fluid through the fluid filter outlet.

In a third aspect, the present disclosure provides a system to recover residual fluid from a soft sided fluid filter having an inlet and an outlet and which is used during processing of a biological fluid by a biological fluid processing system, comprising a reusable hardware unit comprising a fluid filter compression assembly. The fluid filter compression assembly further comprising a housing comprising an outer wall that defines a housing space, at least one bladder positioned within the housing space, at least one conduit, at least one pump, wherein the at least one conduit is in fluid communication with the at least one bladder and the at least one pump, a clamp configured to selectively stop flow through the fluid filter inlet, and wherein the at least one pump is configured to pump fluid media into the at least one bladder.

### Brief Description of the Drawings

In describing the preferred example embodiments, reference is made to the accompanying drawing figures wherein like parts have like reference numerals, and wherein:
FIG. 1 shows an example biological fluid processing system with which the present system and method to recover residual biological fluid from a soft sided fluid filter may be incorporated;
FIG. 2 is an enlarged view of a portion of FIG. 1 showing a portion of the system to recover biological fluid from a soft sided fluid filter;
FIG. 3 is a perspective view of portions of a soft sided fluid filter having an inlet and an outlet spaced above the portion of the portion of the system shown in FIGS. 1 and 2;
FIG. 4 is a partial cross-sectional view of the fluid filter of FIG. 3 spaced above a housing and respective bladders, conduits, and pumps of the example system;
FIG. 5 is a partial cross-sectional view similar to FIG. 4 but with the fluid filter received in the housing between the bladders;
FIG. 6 is a partial cross-sectional view similar to FIG. 5 but schematically showing a clamp engaged to stop flow through the fluid filter inlet and having one of the bladders expanded and partially compressing the fluid filter;
FIG. 7 is a partial cross-section view similar to FIG. 6 but showing both bladders expanded and compressing the fluid filter;
FIG. 8 is a partial cross-sectional view of a second example system to recover biological fluid from a soft sided fluid filter which is similar to the system shown in FIG. 5 but with the fluid filter received in a relatively narrower housing, with a single bladder, conduit, and pump, and the fluid filter received in the housing between the single bladder housing outer wall; and
FIG. 9 is a partial cross-sectional view similar to FIG. 8 but schematically showing the second example with a clamp engaged to stop flow through the fluid filter inlet and having the single bladder expanded and compressing the fluid filter.

It should be understood that the drawings are not to scale. While some mechanical details of the example embodiments, including details of fastening means and other plan and section views of the particular components, may not have been shown, such details are considered to be within the comprehension of those skilled in the art in light of the present disclosure. It also should be understood that the present disclosure and claims are not limited to the preferred embodiments.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

The systems and methods of the present disclosure are described herein in the context of an example apparatus and two alternative example embodiments.

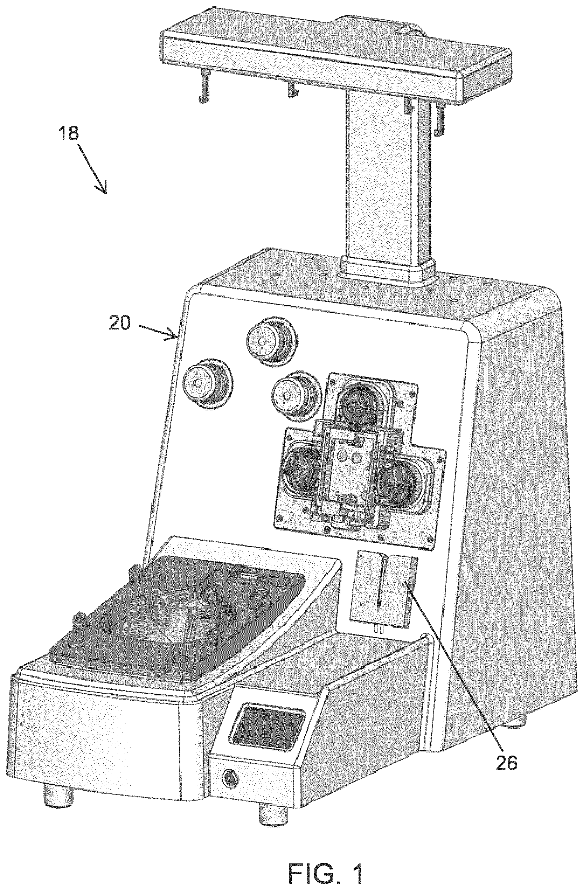

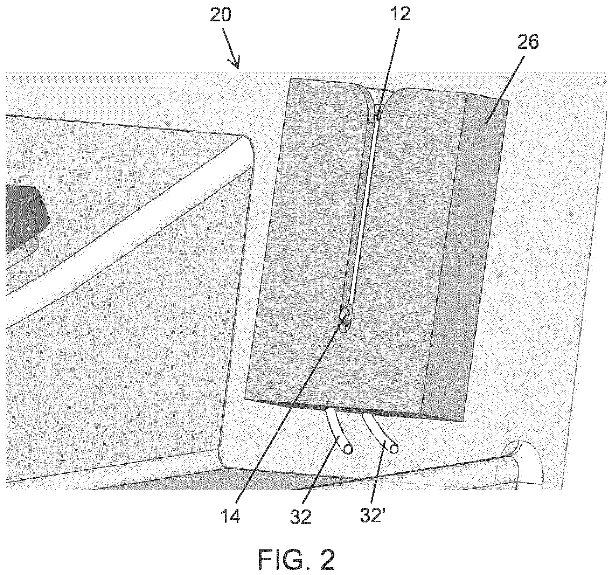

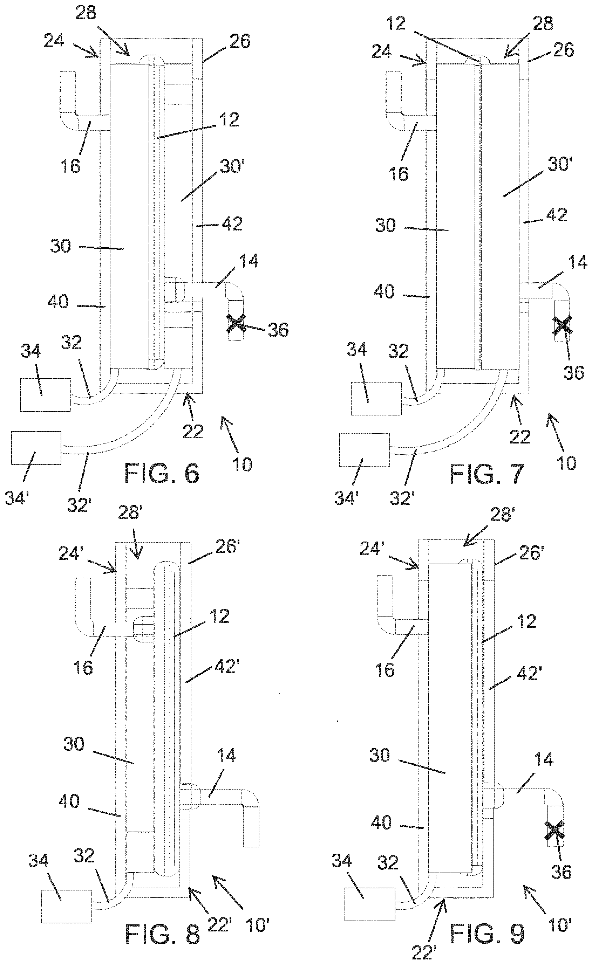

Turning to FIGS. 1-9, two example embodiments of systems and methods for removal of fluid from a biological fluid filter are provided. The first example system 10 is shown in FIGS. 1-7, and a second example system 10' is shown in FIGS. 8-9.

The first example in FIGS. 1-7 provides a system 10 to recover residual biological fluid from a soft sided fluid filter 12 having an inlet 14 and an outlet 16 and which may be used by a biological fluid processing system 18, such as for example the previously mentioned automation system described in International Pub. No. WO 2021/194824. The biological fluid processing system 18 includes a reusable hardware unit 20. The reusable hardware unit 20 includes a fluid filter compression assembly 22. The fluid filter compression assembly 22 further includes a housing 24 having an outer wall 26 that defines a housing space 28 that receives the fluid filter 12, at least one bladder 30, positioned within the housing space 28, at least one conduit 32, and at least one pump 34, wherein the at least one conduit 32 is in fluid communication with the at least one bladder 30 and the at least one pump 34. The fluid filter compression assembly 22 further includes a clamp 36 configured to selectively stop flow through the fluid filter inlet 14. The clamp may be applied manually by an operator or via automation. Similarly, operation of the at least one pump may be triggered manually or as part of automated processing.

The fluid filter 12 is slidably received by the housing 24 within the housing space 28 when the disposable kit is loaded on the reusable hardware unit 20. The housing 24 may be constructed of any suitable relatively rigid material, to provide a stable housing space 26 within which the compressive force of the at least one bladder 30 may be applied to the fluid filter 12. During filtration, the fluid filter 12 may fit relatively tightly between the outer wall 26 of the housing 24 and the at least one bladder 30, or between two bladders 30, 30' within the housing space 28. The at least one bladder 30 or first and second bladders 30, 30' may be unfilled or slightly filled when receiving the fluid filter 12, so as to create optimum compression of the fluid filter 12 following filtration.

The at least one pump 34 is configured to pump fluid media into the at least one bladder 30. When the fluid filter 12 is disposed in the housing space 28 and the at least one pump 34 pumps fluid media into the at least one bladder 30, the fluid filter 12 is compressed. Upon the biological fluid processing system completing filtration, the fluid filter 12 will contain residual biological fluid. When the fluid filter 12 contains residual biological fluid from fluid processing, the clamp 36 is used to selectively stop flow through the fluid filter inlet 14 and the at least one pump 34 pumps fluid media into the at least one bladder 30, at least a portion of the residual biological fluid is expelled through the fluid filter outlet 16 when the fluid filter 12 is compressed. The at least one bladder 30 is disposed between the fixed outer wall 26 of the housing 24 and the fluid filter 12, so when the at least one bladder 30 receives fluid media from the at least one pump 34, the at least one bladder 30 expands and provides compressive force to and ultimately compresses the fluid filter 12.

It will be appreciated that among the various fluid media that may be used, the fluid media may be air and the at least one pump 34 may be an air pump configured to inflate the at least one bladder 30. As such, when the fluid filter 12 is disposed in the housing space 28 and the at least one pump 34 pumps air into the at least one bladder 30, the fluid filter 12 is compressed. Depending on the desired operation, it will be appreciated that compression of the fluid filter 12 may occur a single time or may be cyclical.

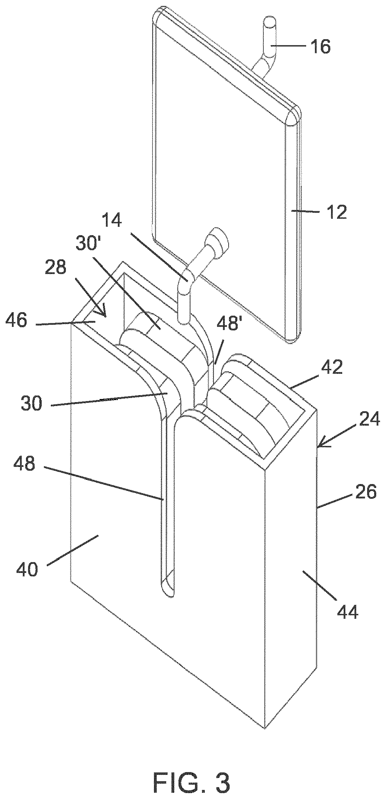

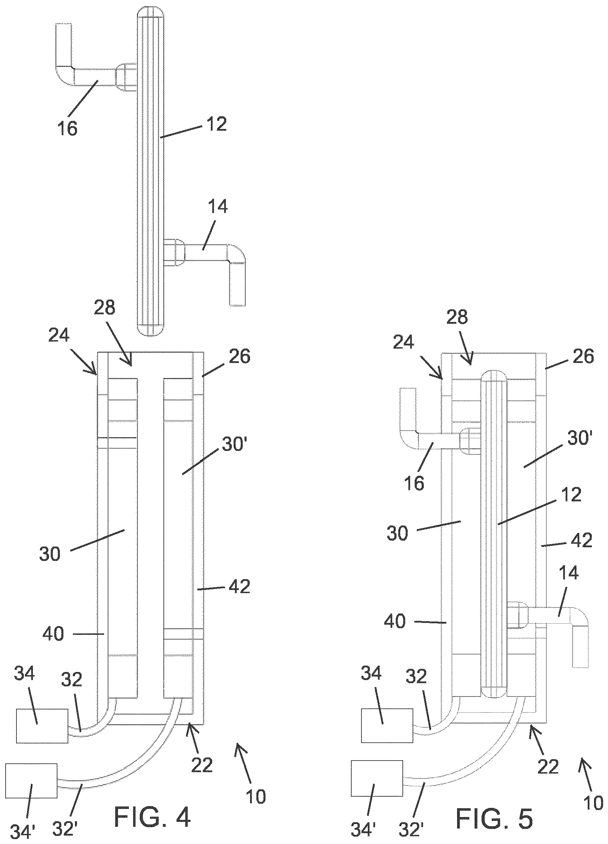

In the system 10, the outer wall 26 of the filter housing 24 may further include opposed front and rear walls 40, 42 and opposed side walls 44, 46 connected to the front and rear walls 40, 42, respectively. In the example shown, the outer wall 26 of the filter housing 24 may further include at least one slot 48 to accommodate the fluid filter inlet 14 or outlet 16. It will be appreciated, as best seen in FIG. 3 and further appreciated in FIGS. 4-7, that with the first example system 10 the outer wall 26 of the filter housing 24 may further include respective slots 48, 48' through the opposed front and rear walls 40, 42. In addition, at least one portion of the outer wall of the housing may be provided by a wall of the biological fluid processing system, such as by adding three walls to a front surface to define the housing space 28. It also will be appreciated that the outer wall could have other than a rectangular shape.

With the first example system 10, as shown in FIGS. 3-7, it will be appreciated that the at least one bladder 30 may include first and second bladders 30, 30' being spaced apart and positioned within the housing space 28. In turn, the at least one conduit 32 may include first and second conduits 32, 32', and the at least one pump 34 may include first and second pumps 34, 34'. With this arrangement, the first conduit 32 is in fluid communication with the first bladder 30 and the first pump 34, and the second conduit 32' is in fluid communication with the second bladder 30' and the second pump 34'. Thus, the first and second pumps 34, 34' are configured to pump fluid media into the first and second bladders 30, 30', respectively.

It will be appreciated from FIGS. 5 and 6 that when the fluid filter 12 is disposed in the housing space 28 between the spaced apart first and second bladders 30, 30' and fluid media is pumped into at least one of the first and second bladders 30, 30' by the respective first or second pump 34, 34', the fluid filter 12 is compressed. In this situation, the fluid filter 12 is at least partially compressed. When the fluid filter 12 contains residual biological fluid from fluid processing, the clamp 36 is used to selectively stop flow through the fluid filter inlet 14 and the at least the first or second pump 34, 34' pumps fluid media into the respective first or second bladder 30, 30', at least a portion of the residual biological fluid is expelled through the fluid filter outlet 16 when the fluid filter 12 is compressed.

To gain more complete compression of the fluid filter 12, it will be appreciated that when the fluid filter 12 is disposed in the housing space 28 between the spaced apart first and second bladders 30, 30' and fluid media is pumped into the first and second bladders 30, 30' by the respective first and second pumps 34, 34', the fluid filter 12 is compressed. As such, when the fluid filter 12 contains residual biological fluid from fluid processing, the clamp 36 is used to selectively stop flow through the fluid filter inlet 14 and the first and second pumps 34, 34' pump fluid media into the respective first and second bladders 30, 30', at least a portion of the residual biological fluid is expelled through the fluid filter outlet 16 when the fluid filter 12 is compressed. As noted previously, while various suitable alternatives may be used, the fluid media may be air and the first and second pumps 34, 34' may be air pumps configured to inflate the respective first and second bladders 30, 30'.

It will be appreciated that FIGS. 8 and 9 show the second example system 10' to recover residual biological fluid from a soft sided fluid filter 12 having an inlet 14 and an outlet 16 and which may be used by a biological fluid processing system 18. Indeed, the system 10' may be incorporated into the same biological fluid processing system 18. However, the second example system 10' does differ in some respects from the first example system 10.

For instance, the second example system 10' includes a reusable hardware unit 20'. The reusable hardware unit 20' includes a fluid filter compression assembly 22'. The fluid filter compression assembly 22' further includes a housing 24' having an outer wall 26' that defines a housing space 28' that receives the fluid filter 12, at least one bladder 30, positioned within the housing space 28', at least one conduit 32, and at least one pump 34, wherein the at least one conduit 32 is in fluid communication with the at least one bladder 30 and the at least one pump 34. The fluid filter compression assembly 22 further includes a clamp 36 configured to selectively stop flow through the fluid filter inlet 14.

Similar to the first example, in the second example system 10', the at least one pump 34 is configured to pump fluid media into the at least one bladder 30. When the fluid filter 12 is disposed in the housing space 28' and the at least one pump 34 pumps fluid media into the at least one bladder 30, the fluid filter 12 is compressed. When the fluid filter 12 contains residual biological fluid from fluid processing, the clamp 36 is used to selectively stop flow through the fluid filter inlet 14 and the at least one pump 34 pumps fluid media into the at least one bladder 30, at least a portion of the residual biological fluid is expelled through the fluid filter outlet 16 when the fluid filter 12 is compressed.

As with the first example, in the second example system 10', it will be appreciated that among the various fluid media that may be used, the fluid media may be, for example, air and the at least one pump 34 may be an air pump configured to inflate the at least one bladder 30. As such, when the fluid filter 12 is disposed in the housing space 28' and the at least one pump 34 pumps air into the at least one bladder 30, the fluid filter 12 is compressed.

The outer wall 26' of the filter housing 24' of the second example has a smaller perimeter, resulting in a smaller housing space 28' and may further include opposed front and rear walls 40', 42' and opposed side walls connected to the front and rear walls 40', 42', respectively. In the second example, as with the first example, the outer wall 26' of the filter housing 24' may further include at least one slot to accommodate the fluid filter inlet 14 or outlet 16, and it will be appreciated in FIGS. 8 and 9 that with the second example system 10' the outer wall 26' of the filter housing 24' may further include respective slots through the opposed front and rear walls 40', 42'.

It will be appreciated that a method of recovering residual biological fluid from a soft sided fluid filter 12 having an inlet 14 and an outlet 16 and which is used by a biological fluid processing system 18 in accordance with either of the above described first and second example systems 10, 10' may be practiced. The method includes passing fluid through the fluid filter inlet 14, the fluid filter 12 and the fluid filter outlet 16, engaging the clamp 36 to selectively stop flow through the fluid filter inlet 14, and engaging the at least one pump 34 to pump fluid media into the at least one bladder 30, causing the fluid filter 12 to be compressed and to expel residual biological fluid through the fluid filter outlet 16. As previously noted, the fluid media may be air and the at least one pump 34 may be an air pump that when engaged inflates the at least one bladder 30.

In practicing the method, as seen in FIGS. 3-7, the at least one bladder 30 may further include first and second bladders 30, 30' being spaced apart and positioned within the housing space 28, the at least one conduit 32 may include first and second conduits 32, 32', at least one pump 34 may include first and second pumps 34, 34'. In this first example configuration, the first conduit 32 is in fluid communication with the first bladder 30 and the first pump 34, and the second conduit 32' is in fluid communication with the second bladder 30' and the second pump 34'. The method further includes engaging the clamp 36 to selectively stop flow through the fluid filter inlet 14, and engaging the first and second pumps 34, 34' to pump fluid media into the respective first and second bladders 30, 30', causing the fluid filter 12 to be compressed and to expel residual biological fluid through the fluid filter outlet 16. As noted among the potential fluid media, the fluid media may be air and the first and second pumps 34, 34' may be air pumps that when engaged inflate the respective first and second bladders 30, 30'.

It will be appreciated that the present disclosure provides first and second example systems 10, 10' to recover residual fluid from a soft sided fluid filter 12 having an inlet 14 and an outlet 16 and which may be used during processing of a biological fluid by a biological fluid processing system 18. The example systems include a reusable hardware unit 20, 20', respectively, including a fluid filter compression assembly 22, 22', respectively. The fluid filter compression assembly 22, 22' further includes a housing 24, 24' respectively, including an outer wall 26, 26' that defines a housing space 28, 28', at least one bladder 30 positioned within the housing space 28, 28', at least one conduit 32, at least one pump 34, wherein the at least one conduit 32 is in fluid communication with the at least one bladder 30 and the at least one pump 34. The fluid compression assembly 22, 22' further includes a clamp 36 configured to selectively stop flow through the fluid filter inlet 14, and wherein the at least one pump 34 is configured to pump fluid media into the at least one bladder 30.

It will be appreciated that with respect to both example systems 10, 10', when the fluid filter 12 is disposed in the housing space 28, 28' and the at least one pump 34 pumps fluid media into the at least one bladder 30, the fluid filter 12 is compressed. Further, when the fluid filter 12 contains residual biological fluid from fluid processing, the clamp 12 is used to selectively stop flow through the fluid filter inlet 14 and the at least one pump 34 pumps fluid media into the at least one bladder 30, at least a portion of the residual biological fluid is expelled through the fluid filter outlet 16 when the fluid filter 12 is compressed.

As previously described, the first example system 10 shows an example which may include first and second bladders 30, 30', first and second conduits 32, 32' and fluid media may be pumped into the first and second bladders 30, 30' by the respective first and second pumps 34, 34'. With the examples shown, either one pump 34 may pump fluid media into one bladder 30 to compress the fluid filter 12, as shown in FIG. 6 with respect to the first example and shown in FIG. 9 with respect to the second example, or at least two pumps 34, 34' may pump fluid media into two respective bladders 30, 30' to compress the fluid filter 12.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

### Other Aspects

Aspect 1. A system to recover residual biological fluid from a soft sided fluid filter having an inlet and an outlet and which is used by a biological fluid processing system, comprising: a reusable hardware unit comprising a fluid filter compression assembly; the fluid filter compression assembly further comprising: a housing comprising an outer wall that defines a housing space that receives the fluid filter; at least one bladder positioned within the housing space; at least one conduit; at least one pump; wherein the at least one conduit is in fluid communication with the at least one bladder and the at least one pump; and a clamp configured to selectively stop flow through the fluid filter inlet.

Aspect 2. The system of Aspect 1, wherein the at least one pump is configured to pump fluid media into the at least one bladder.

Aspect 3. The system of Aspect 1, wherein when the fluid filter is disposed in the housing space and the at least one pump pumps fluid media into the at least one bladder, the fluid filter is compressed.

Aspect 4. The system of Aspect 3, wherein when the fluid filter contains residual biological fluid from fluid processing, the clamp is used to selectively stop flow through the fluid filter inlet and the at least one pump pumps fluid media into the at least one bladder, at least a portion of the residual biological fluid is expelled through the fluid filter outlet when the fluid filter is compressed.

Aspect 5. The system of Aspect 2, wherein the fluid media is air and the at least one pump is an air pump configured to inflate the at least one bladder.

Aspect 6. The system of Aspect 5, wherein when the fluid filter is disposed in the housing space and the at least one pump pumps air into the at least one bladder, the fluid filter is compressed.

Aspect 7. The system of Aspect 1, wherein the outer wall of the filter housing further comprises opposed front and rear walls and opposed side walls connected to the front and rear walls, respectively.

Aspect 8. The system of Aspect 1, wherein the outer wall of the filter housing further comprises at least one slot to accommodate the fluid filter inlet or outlet.

Aspect 9. The system of Aspect 7, wherein the outer wall of the filter housing further comprises respective slots through the opposed front and rear walls.

Aspect 10. The system of Aspect 1, wherein: the at least one bladder further comprises first and second bladders being spaced apart and positioned within the housing space; the at least one conduit further comprises first and second conduits; at least one pump further comprises first and second pumps; and the first conduit is in fluid communication with the first bladder and the first pump, and the second conduit is in fluid communication with the second bladder and the second pump.

Aspect 11. The system of Aspect 10, wherein the first and second pumps are configured to pump fluid media into the first and second bladders, respectively.

Aspect 12. The system of Aspect 10, wherein when the fluid filter is disposed in the housing space between the spaced apart first and second bladders and fluid media is pumped into at least one of the first and second bladders by the respective first or second pump, the fluid filter is compressed.

Aspect 13. The system of Aspect 12, wherein when the fluid filter contains residual biological fluid from fluid processing, the clamp is used to selectively stop flow through the fluid filter inlet and the at least the first or second pump pumps fluid media into the respective first or second bladder, at least a portion of the residual biological fluid is expelled through the fluid filter outlet when the fluid filter is compressed.

Aspect 14. The system of Aspect 10, wherein when the fluid filter is disposed in the housing space between the spaced apart first and second bladders and fluid media is pumped into the first and second bladders by the respective first and second pumps, the fluid filter is compressed. Aspect 15. The system of Aspect 14, wherein when the fluid filter contains residual biological fluid from fluid processing, the clamp is used to selectively stop flow through the fluid filter inlet and the first and second pumps pump fluid media into the respective first and second bladders, at least a portion of the residual biological fluid is expelled through the fluid filter outlet when the fluid filter is compressed.

Aspect 16. The system of Aspect 11, wherein the fluid media is air and the first and second pumps are air pumps configured to inflate the respective first and second bladders.

Aspect 17. A method of recovering residual biological fluid from a soft sided fluid filter having an inlet and an outlet and which is used by a biological fluid processing system, the method comprising: providing a system as set forth in Aspect 1; passing fluid through the fluid filter inlet, the fluid filter and the fluid filter outlet; engaging the clamp to selectively stop flow through the fluid filter inlet; and engaging the at least one pump to pump fluid media into the at least one bladder, causing the fluid filter to be compressed and to expel residual biological fluid through the fluid filter outlet.

Aspect 18. The method of Aspect 17, wherein the fluid media is air and the at least one pump is an air pump that when engaged inflates the at least one bladder.

Aspect 19. The method of Aspect 17, wherein the at least one bladder further comprises first and second bladders being spaced apart and positioned within the housing space, the at least one conduit further comprises first and second conduits, at least one pump further comprises first and second pumps, and the first conduit is in fluid communication with the first bladder and the first pump, and the second conduit is in fluid communication with the second bladder and the second pump, the method further comprising: engaging the clamp to selectively stop flow through the fluid filter inlet; and engaging the first and second pumps to pump fluid media into the respective first and second bladders, causing the fluid filter to be compressed and to expel residual biological fluid through the fluid filter outlet.

Aspect 20. The method of Aspect 19, wherein the fluid media is air and the first and second pumps are air pumps that when engaged inflate the respective first and second bladders.

Aspect 21. A system to recover residual fluid from a soft sided fluid filter having an inlet and an outlet and which is used during processing of a biological fluid by a biological fluid processing system, comprising: a reusable hardware unit comprising a fluid filter compression assembly; the fluid filter compression assembly further comprising: a housing comprising an outer wall that defines a housing space; at least one bladder positioned within the housing space; at least one conduit; at least one pump; wherein the at least one conduit is in fluid communication with the at least one bladder and the at least one pump; a clamp configured to selectively stop flow through the fluid filter inlet; and wherein the at least one pump is configured to pump fluid media into the at least one bladder.

Aspect 22. The system of Aspect 21, wherein when the fluid filter is disposed in the housing space and the at least one pump pumps fluid media into the at least one bladder, the fluid filter is compressed.

Aspect 23. The system of Aspect 22, wherein when the fluid filter contains residual biological fluid from fluid processing, the clamp is used to selectively stop flow through the fluid filter inlet and the at least one pump pumps fluid media into the at least one bladder, at least a portion of the residual biological fluid is expelled through the fluid filter outlet when the fluid filter is compressed.

## Claims

1. A system to recover residual biological fluid from a soft sided fluid filter having an inlet and an outlet and which is used by a biological fluid processing system, comprising:
a reusable hardware unit comprising a fluid filter compression assembly;
the fluid filter compression assembly further comprising:
a housing comprising an outer wall that defines a housing space that receives the fluid filter;
at least one bladder positioned within the housing space;
at least one conduit;
at least one pump;
wherein the at least one conduit is in fluid communication with the at least one bladder and the at least one pump; and
a clamp configured to selectively stop flow through the fluid filter inlet.

2. The system of claim 1, wherein the at least one pump is configured to pump fluid media into the at least one bladder.

3. The system of claim 1, wherein when the fluid filter is disposed in the housing space and the at least one pump pumps fluid media into the at least one bladder, the fluid filter is compressed.

4. The system of claim 3, wherein when the fluid filter contains residual biological fluid from fluid processing, the clamp is used to selectively stop flow through the fluid filter inlet and the at least one pump pumps fluid media into the at least one bladder, at least a portion of the residual biological fluid is expelled through the fluid filter outlet when the fluid filter is compressed.

5. The system of claim 2, wherein the fluid media is air and the at least one pump is an air pump configured to inflate the at least one bladder.

6. The system of claim 5, wherein when the fluid filter is disposed in the housing space and the at least one pump pumps air into the at least one bladder, the fluid filter is compressed.

7. The system of claim 1, wherein the outer wall of the filter housing further comprises opposed front and rear walls and opposed side walls connected to the front and rear walls, respectively.

8. The system of claim 1, wherein the outer wall of the filter housing further comprises at least one slot to accommodate the fluid filter inlet or outlet.

9. The system of claim 7, wherein the outer wall of the filter housing further comprises respective slots through the opposed front and rear walls.

10. The system of claim 1, wherein:
the at least one bladder further comprises first and second bladders being spaced apart and positioned within the housing space;
the at least one conduit further comprises first and second conduits;
at least one pump further comprises first and second pumps; and
the first conduit is in fluid communication with the first bladder and the first pump, and the second conduit is in fluid communication with the second bladder and the second pump.

11. The system of claim 10, wherein the first and second pumps are configured to pump fluid media into the first and second bladders, respectively.

12. The system of claim 10, wherein when the fluid filter is disposed in the housing space between the spaced apart first and second bladders and fluid media is pumped into at least one of the first and second bladders by the respective first or second pump, the fluid filter is compressed.

13. The system of claim 12, wherein when the fluid filter contains residual biological fluid from fluid processing, the clamp is used to selectively stop flow through the fluid filter inlet and the at least the first or second pump pumps fluid media into the respective first or second bladder, at least a portion of the residual biological fluid is expelled through the fluid filter outlet when the fluid filter is compressed.

14. The system of claim 10, wherein when the fluid filter is disposed in the housing space between the spaced apart first and second bladders and fluid media is pumped into the first and second bladders by the respective first and second pumps, the fluid filter is compressed.

15. The system of claim 14, wherein when the fluid filter contains residual biological fluid from fluid processing, the clamp is used to selectively stop flow through the fluid filter inlet and the first and second pumps pump fluid media into the respective first and second bladders, at least a portion of the residual biological fluid is expelled through the fluid filter outlet when the fluid filter is compressed.
